# EUROPEAN PATENT SPECIFICATION

(11) **EP 3 903 872 B1**
(45) Date of publication and mention of the grant of the patent: **05.06.2024**
(21) Application number: 19901652.8
(22) Date of filing: 19.09.2019
(51) Int. Cl.: A61M 25/14

(54) **BENDABLE CATHETER**
BIEGBARER KATHETER
CATHÉTER SOUPLE

(30) Priority: 29.12.2018 CN 201811653891; 29.12.2018 CN 201822272379 U
(43) Date of publication of application: 03.11.2021
(73) Proprietor: Hangzhou Endonom Medtech Co., Ltd, Hangzhou, Zhejiang 310051 (CN)
(72) Inventor: ZHANG, Tingchao, Hangzhou, Zhejiang 310051 (CN); JIANG, Quanjie, Hangzhou, Zhejiang 310051 (CN); HUANG, Qiao, Hangzhou, Zhejiang 310051 (CN)
(74) Representative: Hamer, Christopher K.
(86) International application number: PCT/CN2019/106738
(87) International publication number: WO 2020/134219

(56) References cited:
- WO-A1-2017/155892
- US-B2- 10 278 852

## Description

### TECHNICAL FIELD

The disclosure relates to the technical field of medical equipment, in particular to a bendable catheter.

### BACKGROUND

In recent years, interventional operation has rapidly emerged and promoted due to less damage to a human body and a short operation time. As auxiliary equipment for the interventional operation, catheters are mainly used to establish a passage between human blood vessels and the external so as to deliver diagnostic and/or therapeutic devices.

In order to adapt to individual differences in a human physiological anatomical structure, bendable catheters have been widely used. The bendable catheter includes a bendable section that is arranged at a distal end of a tube body of the catheter. A handle of the catheter is operated to drive a traction wire connected to the bendable section to axially move so that the distal end of the tube body is repeatedly bent at different angles until a bending angle accords with specific physiological structure characteristics of lumens of the human body. The bending angle is then locked, the distal end of the tube body is aligned to an inlet of a target lumen (such as a certain blood vessel), and the diagnostic and/or therapeutic device is delivered to the target lumen through the tube body.

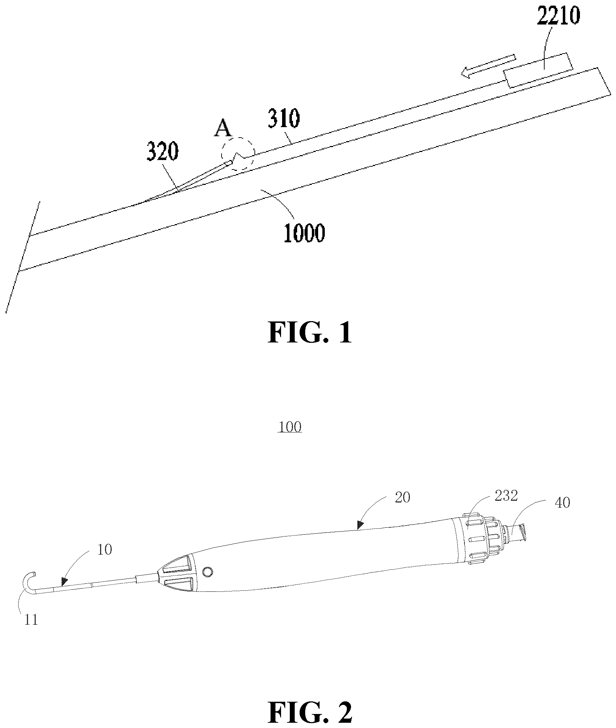

Referring to FIG. 1, in a bendable catheter in the related art, a traction wire is generally disposed in a threading tube 320, most of the threading tube 320 is embedded in a wall of a tube body 1000, a proximal end penetrates out of the wall of the tube body 1000, and particularly the proximal end of the traction wire 310 penetrates out of the threading tube 320 to be connected to a slide block 2210 in a handle, the slide block 2210 moves to drive the traction wire 310 to axially move, so as to bend a bendable section at a distal end of the tube body 1000. Since the traction wire 310 is generally very thin, a process of introducing the traction wire 310 to the threading tube 320 may lag behind a moving process of the slide block 2210 under conditions of strong push force and over-high push speed when the traction wire 310 is pushed to move to the distal end of the tube body 1000 by the slide block 2210, so that the traction wire 310 is easy to be severely bent or even broken near an proximal opening of the threading tube 320 (as position A in FIG. 1), which hinders implementation of a bend adjusting function of the bendable catheter.

US 7678074 B2 relates to a deflectable catheter. The deflectable catheter includes a catheter shaft having a deflectable distal tip. A support member is coupled around a proximal portion of the catheter shaft, and the support member includes a first brake portion extending along at least a portion of the support member. A handle is coupled around the support member. The deflectable catheter includes a carriage moveably coupled along the handle, and the carriage includes a second brake portion sized and shaped to engage with at least a portion of the first brake portion. A flexible element is coupled between the deflectable distal tip and the carriage. A biasing device is adapted to bias the second brake portion into engagement with the first brake portion.

CN 107206210 A relates to a steerable catheter device. The steerable catheter device includes a shaft comprising a proximal portion, a distal portion, and a pull-wire lumen that extends at least partially through the proximal and distal portions. A pull wire extends through the pull-wire lumen and has a proximal end portion and a distal end portion, wherein the distal end portion of pull wire is fixed to the distal end portion of the shaft. An adjustment mechanism is operatively connected to the proximal end portion of the pull wire and configured to increase and decrease tension in the pull wire to adjust the curvature of the distal portion of the shaft. An axially non-compressible pull-wire sleeve extends co-axially through the pull-wire lumen and over the pull wire.

WO 2017/155892 A1 relates to a steerable catheter 100. The steerable catheter 100 includes a tubular sheath 102 having a longitudinally-extending lumen formed within a wall thereof and a steering mechanism 120 disposed within the longitudinally-extending lumen. The steering mechanism includes a pull tube 122 and a pull wire 132. At least a segment of the pull wire is coaxially disposed within the pull tube. The pull tube has a distal end attached at a first anchor point to the tubular sheath, and the pull wire has a distal end attached at a second anchor point to the tubular sheath. The second anchor point is distal of the first anchor point. The pull tube and the pull wire are separately tensioned to bend respective regions of the tubular sheath.

### SUMMARY

The disclosure provides a bendable catheter, which can prevent a traction wire from being severely bent or broken near a proximal end of a threading tube to ensure smooth implementation of a bend adjusting function of the bendable catheter. The invention is specified by the independent claim. Further embodiments are specified in the dependent claims.

In order to achieve the above objective, a bendable catheter is provided and includes a tube body and a traction member. The tube body includes a bendable section arranged at a distal end of the tube body. The traction member includes a traction wire, a threading tube, a protective casing, and a clamping tube. The traction wire has a distal end connected to the bendable section, a proximal end wrapped and fixed within the clamping tube, and a remainder which is sequentially threaded through the threading tube and the protective casing in a direction from the distal end of the tube body to a proximal end of the tube body. The proximal end of the threading tube is relatively fixed with a distal end of the protective casing without a gap in axial directions of the threading tube and the protective casing. A distal end of the clamping tube is movably threaded in the protective casing. The clamping tube is operable to move in the protective casing to drive the traction wire to move in the protective casing and the threading tube, so as to bend or straighten the bendable section.

In an implementation, the proximal end of the threading tube and the distal end of the protective casing are in abutting joint, or the proximal end of the threading tube is threaded in the distal end of the protective casing.

In an implementation, the protective casing includes an inclined section, a transition section, and a straight section connected in sequence. The transition section is smoothly connected with the inclined section and the straight section. The inclined section is relatively fixed with the proximal end of the threading tube. The proximal end of the clamping tube is movably threaded in the straight section.

In an implementation, an inside diameter of the protective casing is 0.1 mm to 0.3 mm greater than an outside diameter of the clamping tube, and is 0.3 mm to 0.6 mm greater than a diameter of the traction wire.

In an implementation, a length of the protective casing is greater than a distance that the traction wire moves to drive the bendable section to switch between a straight state and a state with a maximum bending angle.

In an implementation, the proximal end of the threading tube extends out of the tube body, and a remainder thereof is embedded in a wall of the tube body.

In an implementation, the bendable catheter further includes a bend adjusting handle. The bend adjusting handle is connected to the proximal end of the tube body as well as the proximal end of the clamping tube, and is configured to control the clamping tube to move in the protective casing.

In an implementation, the bend adjusting handle includes a fixing base, a drive mechanism, and a drive control mechanism. The proximal end of the tube body is threaded and fixed in the fixing base. The protective casing is fixed relative to the fixing base. The clamping tube is connected to the drive mechanism. The drive control mechanism is connected to the drive mechanism and configured to control the drive mechanism to move relative to the fixing base. The drive mechanism is configured to move to drive the clamping tube to move in the protective casing.

In an implementation, the drive mechanism includes a slide block and a fixed block fixed to the slide block. The clamping tube is fixed to the fixed block. The slide block defines a threaded groove on a surface of the slide block. The drive control mechanism includes a rotary sleeve. The rotary sleeve is provided with a thread engaged with the threaded groove on an inside of the rotary sleeve. The rotary sleeve is rotated to drive the slide block to move relative to the fixing base.

In an implementation, the length of the protective casing is greater than a maximum stroke of the slide block.

In an implementation, the fixing base defines an axial slot axially extended along the fixing base. The slide block is movably disposed in the axial slot. The rotary sleeve is configured to rotate to drive the slide block to move along the axial slot.

In an implementation, the proximal end of the tube body is embedded in the fixing base. A central axis of the tube body coincides with a central axis of the fixing base. The fixing base defines an inclined slot. The inclined slot has one end extended to the tube body and the other end extended to the axial slot, and the protective casing is fixed in the inclined slot.

The bendable catheter is provided. The clamping tube is configured to wrap and fix the traction wire. The protective casing is disposed between the threading tube and the clamping tube. There is no gap, in axial directions of the threading tube and the protective casing, between the proximal end of the threading tube and the distal end of the protective casing. The distal end of the clamping tube is movably threaded in the protective casing, so that a part, between the threading tube and the clamping tube, of the traction wire is threaded in the protective casing. In the process of bending or straightening the bendable section of the tube body, the clamping tube drives the traction wire to move in the protective casing and the threading tube, so that the part, between the threading tube and the clamping tube, of the traction wire is remained in the protective casing. An inner cavity of the protective casing limits a degree of bending deformation of the traction wire, and can prevent the traction wire from being severely bent or broken near the proximal end of the threading tube, thereby ensuring smooth implementation of the bend adjusting function of the bendable catheter.

### BRIEF DESCRIPTION OF THE DRAWINGS

In order to describe the technical solutions in this implementations of the disclosure or a related art more clearly, the drawings required to be used in descriptions about the implementations or the related art will be simply introduced below. It is apparent that the drawings in the following descriptions are only some implementations of the disclosure. Those of ordinary skill in the art may further obtain other drawings according to these drawings without creative work.
FIG. 1 is a schematic diagram illustrated severe bending of a traction wire in a bendable catheter in a related art.
FIG. 2 is a three-dimensional structure schematic view of a bendable catheter according to an implementation of the disclosure.
FIG. 3 is an exploded view of the bendable catheter according to an implementation of the disclosure.
FIG. 4 is a cross sectional view of the bendable catheter according to an implementation of the disclosure.
FIG. 5 is a partial enlarged view of Circle II in FIG. 4.
FIG. 6 is a structural schematic view of distal ends of a traction member and a tube body of the bendable catheter according to an implementation of the disclosure.
FIG. 7 is a schematic view illustrating position states of a traction wire, a threading tube, a protective casing, and a clamping tube when the bendable section of the bendable catheter according an implementation of the disclosure is at a maximum bending angle.
FIG. 8 is schematic diagram view illustrating position states of the traction wire, the threading tube, the protective casing, and the clamping tube when the bendable section of the bendable catheter according to an implementation of the disclosure is at a straight state.
FIG. 9 is a structural schematic view of proximal ends of the traction member and the tube body of the bendable catheter according to an implementation of the disclosure.
FIG. 10 is a structural schematic view of a protective casing of the bendable catheter according to an implementation of the disclosure.

### DETAILED DESCRIPTION

The technical solutions in this implementations of the disclosure will be described below in combination with the drawings in this implementations of the disclosure. It is apparent that the described implementations are not all, but part of implementations of the disclosure. All other implementations obtained by those of ordinary skill in the art on the basis of the implementations in the disclosure without creative work shall fall within the scope of protection of the disclosure.

In order to describe structures of a bend adjusting handle and a bendable catheter more clearly, terms "proximal end" and "distal end" are defined here as commonly used terms in the field of interventional medical treatment. Specifically, "distal end" indicates one end away from an operator during a surgical operation, and "proximal end" indicates one end close to the operator during the surgical operation.

Unless otherwise defined, all technical and scientific terms used in the disclosure have the same meaning as commonly understood by those skilled in the art. The terms used in the specification of the disclosure herein are only for the purpose of describing the specific implementations, and are not intended to limit the disclosure.

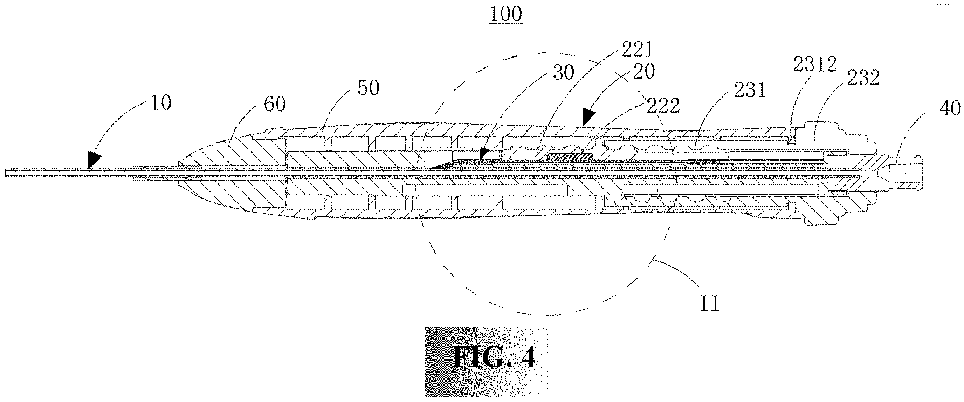

Referring to FIGS. 2 to 5, a bendable catheter 100 is provided. The bendable catheter 100 includes a tube body 10, a bend adjusting handle 20, and a traction member 30. The bend adjusting handle 20 is connected to a proximal end of the tube body 10. The tube body 10 includes a bendable section 11 disposed at a distal end of the tube body 10. A bending angle of the bendable section 11 may be adjusted according to actual requirements. In this implementation, the tube body 10 is provided with a single bendable section 11. It can be understood that in other implementations of the disclosure, the tube body 10 may also be provided with multiple bendable sections 11.

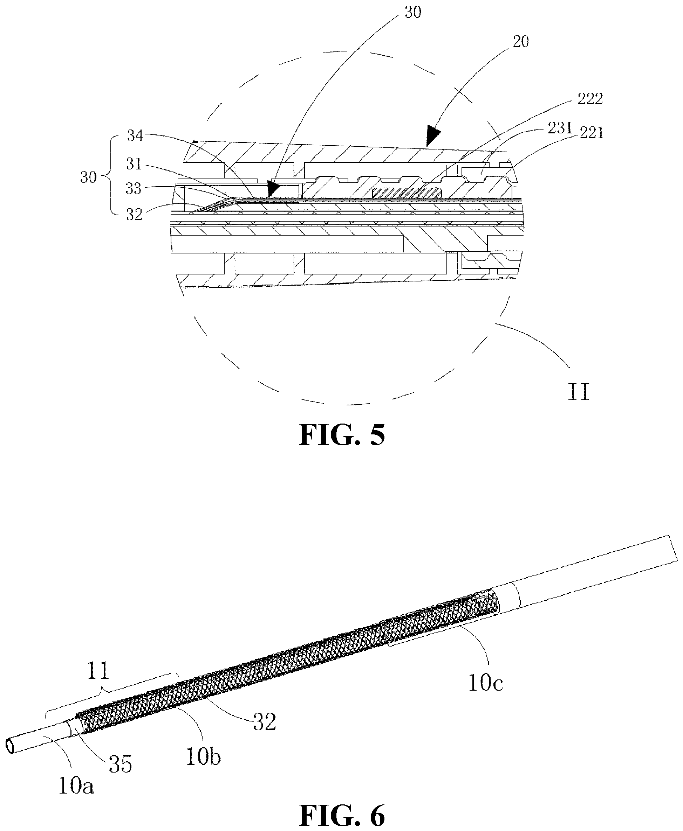

Referring to FIG. 2 and FIG. 6, the tube body 10 includes an inner film 10a, a reinforced tube 10b sleeved on the inner film 10a, and an outer tube 10c sleeved on the reinforced tube 10b. In this implementation, the inner film 10a is a flexible tube made of a flexible material such as Polytetrafluoroethylene (PTFE), which is easy to be bent. The reinforced tube 10b is of a metal braided mesh structure, which has a certain rigidity and can be bent axially to provide a support for the tube body 10, thereby preventing the tube body 10 from radially torsional deformation, without affecting bending of the bendable section 11 of the tube body 10. The outer tube 10c is made of a material with a certain hardness, such as PEBAX, for protecting the tube body 10. In addition, the hardness of a part of the outer tube 10c corresponding to the bendable section 11 is less than the hardness of other part of the outer tube 10c, so that protection to the tube body 10 is achieved while affection to bending of the bendable section 11 is avoided. In this implementation, the hardness of the part, made of the PEBAX material, of the outer tube 10c corresponding to the bendable section 11 is less than the hardness of other part of the outer tube 10c. Further, in this implementation, the inner film 10a, the reinforced tube 10b, and the outer tube 10c are composited and molded by means of hot melting to form a delivery cavity fully running through the distal end from the proximal end. It can be understood that in other implementations of the disclosure, in the case of meeting usage requirements, the tube body 10 may only include the inner film 10a and/or the outer tube 10c. The inner film 10a, the reinforced tube 10b, and the outer tube 10c may be made of other materials except this implementation.

Further, in some implementations of the disclosure, the distal end of the tube body 10 is a curved end with smooth surface, namely a Tip end. A radiopaque developing ring (not illustrated), such as a tantalum ring, is arranged close to the Tip end, so that whether the distal end of the tube body 100 reaches a specified position can be accurately known through a developing apparatus.

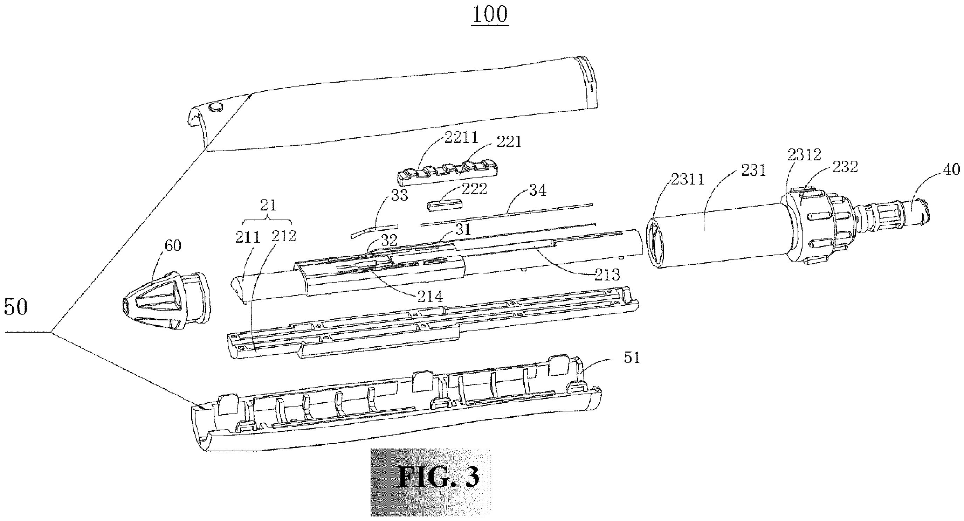

Referring to FIGS. 3 to 9, the traction member 30 includes a traction wire 31, a threading tube 32, a protective casing 33, and a clamping tube 34. The traction wire 31 has a distal end connected to the bendable section 11, a proximal end wrapped and fixed within the clamping tube 34, and a remainder which is sequentially movably threaded in the threading tube 32 and the protective casing 33 in a direction from the distal end of the tube body to a proximal end of the tube body.

The traction wire 31 is used to drive the bendable section 11 to be bent or straightened. The traction wire 31 has a certain strength. In this implementation, the traction wire 31 is a single wire. Alternatively, the traction wire 31 is a strand of wires. The cross section of the traction wire 31 may be in various shapes such as a circle, which is not limited herein. On the basis that the traction wire 31 has a certain strength to implement a traction function, the radial section of the traction wire 31 can be as small as possible. Therefore, a preferred range of a diameter of the traction wire 31 is 0.05 mm to 0.25 mm. The traction wire 31 may be made of a metal material, such as stainless steel, tungsten alloy, cobalt-chromium alloy, or nickel-titanium alloy, may also be made of high polymer with a certain strength, which is not limited herein. In this implementation, the traction wire 31 is preferably a stainless steel wire with a diameter of 0.25 mm.

An anchoring ring 35 is arranged at one end, connected to the bendable section 11, of the traction wire 31. The anchoring ring 35 is a ring part sleeved on the bendable section 11. In other words, the distal end of the traction wire 31 is connected to the bendable section 11 through the anchoring ring 35. In this implementation, the anchoring ring 35 is sleeved on a position, corresponding to the bendable section 11, of the inner film 10a. A contact area between the traction member 30 and the bendable section 11 of the tube body 10 is increased by means of the anchoring ring 35, so that traction on bending of the bendable section 11 may be better achieved. The anchoring ring 35 may be made of a metal material or a high-polymer material. In this implementation, the anchoring ring 35 is made of metal such as SUS304 stainless steel. A connection mode of the traction wire 31 and the anchoring ring 35 includes, but is not limited to, bonding, welding, hot melting, knotting, and the like.

Most of the traction wire 31 is movably threaded in the threading tube 32 so that a traction direction of the traction wire 31 is limited by the threading tube 32, and the traction wire 31 is protected by the threading tube 32. Most of the threading tube 32 is embedded in a wall of the tube body 10, and extends along an axial direction of the tube body 10, so that the traction wire 31 threaded in the threading tube 32 may move along the axial direction of the tube body 10 when moving in the threading tube 32. The proximal end of the threading tube 32 extends out of the wall of the tube body 10 at a specific angle, so that the traction wire 31 threaded therein can penetrate out the threading tube 32 at a specific angle. In this implementation, the threading tube 32 is embedded between the inner film 10a and the reinforced tube 10b. Since the threading tube 32 is embedded in the tube body 10, a diameter of the tube body 10 can be reduced to ensure that the tube body 10 easily moves in a target lumen of the human body. The threading tube 32 may be made of various materials applicable to medical apparatus and equipment, such as metal or high polymer. An appropriate gap is reserved between an inside diameter of the threading tube 32 and the diameter of the traction wire 31 so that the traction wire 31 may smoothly move within the threading tube 32. In this implementation, a difference value between the inside diameter of the threading tube 32 and the diameter of the traction wire 31 is greater than 0 and less than 0.1 mm.

A proximal end of the clamping tube 34 is connected to the bend adjusting handle 20, so that the proximal end of the traction wire 31 is connected to the bend adjusting handle 20 via the clamping tube 34, thereby avoiding a risk of breakage when the traction wire 31 is directly connected to the bend adjusting handle 20 due to thinness of the traction wire 31, and increasing a connection strength between the traction wire 31 and the bend adjusting handle 20. In addition, a distal end of the clamping tube 34 is movably threaded in the protective casing 33, that is, the bend adjusting handle 20 drives the clamping tube 34 and the traction wire 31 to move to adjust a bending state of the bendable section 11, and thus the distal end of the clamping tube 34 is remained in the protective casing 33. It is noted that, the proximal end of the threading tube 32 is relatively fixed with the distal end of the protective casing 33 without a gap in axial directions of the threading tube and the protective casing. "Relatively fixed" means the proximal end of the threading tube 32 is fixed with the distal end of the protective casing 33 are fixed by welding, bonding or the like, or through a fastener. Alternatively, the threading tube 32 and the protective casing 33 may also be respectively fixed, to prevent the proximal end of the threading tube 32 and the distal end of the protective casing 33 from relative movement.

In this implementation, the proximal end of the threading tube 32 is wrapped and fixed in the distal end of the protective casing 33. It can be understood that the proximal end of the threading tube 32 and the distal end of the protective casing 33 may also be in abutting joint. In other words, a tip of the distal end of the protective casing 33 and a tip of the proximal end of the threading tube 32 are in end-to-end joint and fixed together. Alternatively, the protective casing 33 may be formed by extending from the proximal end of the threading tube 32, that is, the protective casing 33 and the threading tube 32 are of an integral structure.

Since the distal end of the clamping tube 34 is movably threaded in the protective casing 33, an inside diameter of the protective casing 33 cannot hinder smooth movement of the clamping tube 34 in the protective casing 33, and a small gap is required to be reserved between the protective casing 33 and the traction wire 31 to reduce radial moving space of the traction wire 31 so as to limit a degree of bending of the traction wire 31 and prevent the traction wire from excessive bending. Preferably, the inside diameter of the protective casing 33 is 0.1 mm to 0.3 mm greater than an outside diameter of the clamping tube 34, and the inside diameter of the protective casing 33 is 0.3 mm to 0.6 mm greater than the diameter of the traction wire 31.

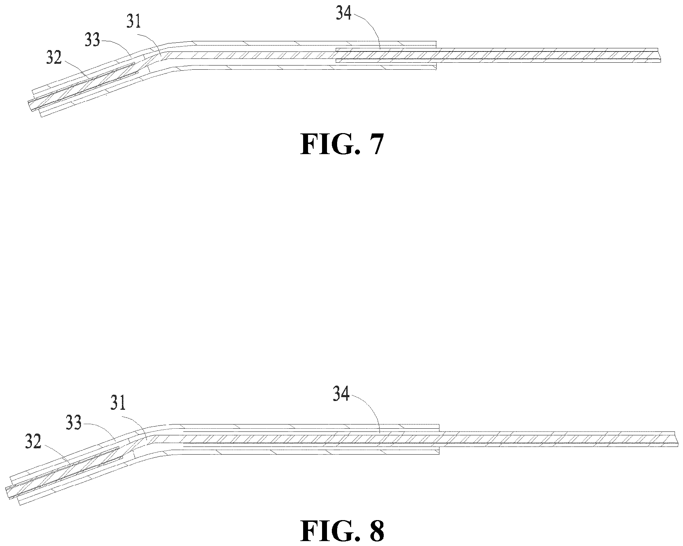

Referring to FIG. 7 and FIG. 8, a length of the protective casing 33 is greater than a distance that the traction wire 31 moves to drive the bendable section 11 of the tube body 10 to adjust to a state with a maximum bending angle from an initial straight state. As such, the distal end of the clamping tube 34 is remained in the protective casing 33. A section of the traction wire 31, exposed between the threading tube 32 and the clamping tube 34 is wrapped within the protective casing 33 all the time. An inner cavity of the protective casing 33 may always limit the degree of bending of the section of traction wire 31. In addition, once the section of traction wire 31 is bent to touch the inner cavity of the protective casing 33, the inner cavity of the protective casing 33 may apply a pushing force to the bending traction force 31 to drive the traction wire 31 to thread the threading tube 32. In this way, the section of traction wire 31 restores to a relatively straight state, so that the risk of severe bending or breakage of the traction wire 31 near the proximal end of the threading tube 32 can be reduced or even avoided, ensuring that the bendable section 11 of the tube body 10 can be smoothly bent or straightened. In this implementation, the protective casing 33 and the traction wire 31 are consistent in section shapes, and the protective casing 33 is preferably made of metal such as stainless steel, red copper, and aluminum alloy. It can be understood that in other implementations, the protective casing 33 may also be made of high polymer with a certain strength. Preferably, in this implementation, the protective casing 33 is a stainless tube with an inside diameter of 0.7 mm and an outside diameter of 1.0 mm.

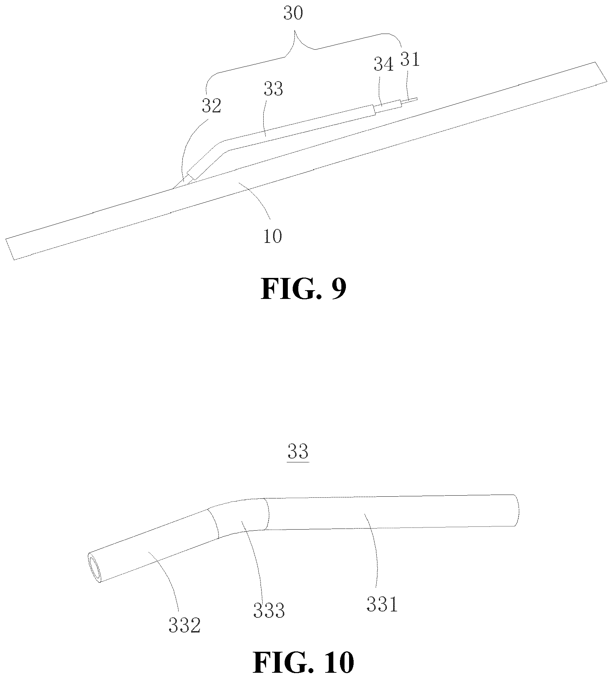

Further, referring to FIG. 9 and FIG. 10, in this implementation, the protective casing 33 includes a straight section 331 axially parallel to the tube body 10, an inclined section 332, and a transition section 333 connected between the straight section 331 and the inclined section 332. The inclined section 332 has the same angle of inclination as a part, extended out of the wall of the tube body 10, of the threading tube 32. The transition section 333 is curved, and smoothly connected with the inclined section 332 and the straight section 331 to ensure that the traction wire 31 may smoothly move in the protective casing 33. The inclined section 332 and a proximal end of the part, extended out of the wall of the tube body 10, of the threading tube 32 are in relative fixing and have the same angle of inclination, so that hindering to the movement of the traction wire 31 within the protective casing 33 may be avoided. The clamping tube 34 is movably threaded in the straight section 331. A length of the straight section 331 is greater than a moving distance of the traction wire 31 in the process that the bendable section 11 is adjusted to a state with the maximum bending angle from the initial straight state, so that the distal end of the clamping tube 34 is remained in the protective casing 33.

Referring back to FIGS. 2 to 5, the bend adjusting handle 20 includes a fixing base 21, a drive mechanism, and a drive control mechanism. The proximal end of the tube body 10 is fixed to the fixing base 21. The protective casing 33 is preferably fixed to the fixing base 21. The clamping tube 34 is connected to the drive mechanism. The drive control mechanism is connected to the drive mechanism and configured to control the drive mechanism to move relative to the fixing base 21. The drive mechanism is configured to move to drive the clamping tube 34 to move in the protective casing 33, so as to drive the traction wire 31 to move to adjust a bending state of the bendable section 11. The fixing base 21 is substantially a cylinder, and the tube body 10 is threaded in the center of the fixing base 21 and fixed to the fixing base 21. In this implementation, the fixing base 21 is divided into a first part 211 and a second part 212 which are fastened mutually. The first part 211 and the second part 212 define corresponding grooves which cooperate to form a center hole for threading of the tube body 10. Thus, the tube body 10 can be conveniently threaded in the fixing base 21 and fixed to the same. Further, the fixing base 21 defines an axial slot 213 extending along an axial direction of the fixing base 21, and an inclined slot 214 extending to the axial slot 213 from the tube body 10. In an implementation, the inclined slot 214 includes an inclined zone and a straight zone connected to the inclined zone. The inclined zone has one end connected to the straight zone and the other end extended to tube body 10, so that the threading tube 32 extending out of the wall of the tube body 10 extends into the inclined zone. The straight zone has one end connected to the inclined zone and the other end extended to the axial slot 213. In this implementation, the inclined zone and an inclined section 332 of the protective casing 33 have the same angle of inclination. The inclined section 332 and the part, extending out of the wall of the tube body 10, of the threading tube 32 are fixed in the inclined zone preferably by means of gluing, and the straight section 331 of the protective casing 33 is fixed in the straight zone preferably by means of gluing, so that the protective casing 33 can be stably fixed in the bendable catheter 100.

The drive mechanism includes a slide block 221 and a fixed block 222 fixed to the slide block 221. In this implementation, the slide block 221 defines a groove. The fixed block 222 is inserted and fixed in the groove. The proximal end of the clamping tube 34 is fixed to the fixed block 222. In this implementation, the clamping tube 34 and the fixed block 222 are preferably fixed by means of welding. It can be understood that in other implementations of the disclosure, the clamping tube 34 and the fixed block 222 may also be fixed by means such as gluing. The slide block 221 is movably disposed in the axial slot 213. The slide block 221 defines a threaded groove 2211 on a surface of the slide block 221. The drive control mechanism includes a rotary sleeve 231. The rotary sleeve 231 is provided with a thread 2311 engaged with the threaded groove 2211 on an inside of the rotary sleeve. With engagement between the thread 2311 and the threaded groove 2211, the rotary sleeve 231 may be rotated to drive the slide block 221 to move in the axial slot 213 along the axial direction of the fixing base 21. In the disclosure, the clamping tube 34 is fixed to the fixed block 222, the slide block 221 moves in the axial slot 213 can drive the clamping tube 34 to move in the protective casing 33, and the traction wire 31 fixed to the clamping tube 34 then pull the bendable section 11 to be bent or straightened. Further, the drive control mechanism further includes a rotary grip 232 on the peripheral surface of a proximal end of the rotary sleeve 231. Anti-slip structures are arranged on the outer surface of the rotary grip 232, so that the rotary sleeve 231 can be rotated more easily by rotating the rotary grip 232.

Further, a Luer taper 40 is fixed at the proximal end of the rotary sleeve 231, the tube body 10 extends through the fixing base 21 to be connected to the Luer taper 40, so that a diagnostic and/or therapeutic device may enter the target lumen of the human body through the Luer taper 40 and the tube body 10, sequentially.

Further, the bend adjusting handle 20 further includes a housing 50 that protects structures therein. The fixing base 21 and the drive mechanism are both accommodated in the housing 50, the distal end of the rotary sleeve 231 is accommodated in the housing 50, and is rotationally connected to the housing 50, and the rotary grip 232 is arranged outside the housing 50 to facilitate a rotating operation. In an implementation, a clamp ring 51 is arranged on an inner wall of a proximal end of the housing 50, a clamp groove 2312 is circularly formed on the peripheral surface of the rotary sleeve 231, and the clamp ring 51 is clamped in the clamp groove 2312, so that a rotational connection between the rotary sleeve 231 and the housing 50 is achieved. Further, in this implementation, the housing 50 includes two parts that are detachably connected, so that the fixing base 21, the drive mechanism, and the like may be fixed in the housing 50 more easily.

Further, in the disclosure, the bend adjusting handle 20 further includes an end cap 60 fixed to a distal end of the housing 50, and the tube body 10 extends through the end cap 60 to enter the fixing base 21.

The bendable catheter 100 is provided. The clamping tube 34 is configured to wrap and fix the traction wire 31, and the protective casing 33 is disposed between the threading tube 32 and the clamping tube 34. The proximal end of the threading tube 32 is relatively fixed with the distal end of the protective casing 33 without a gap in axial directions of the threading tube and the protective casing. The distal end of the clamping tube 34 is movably threaded in the protective casing 33, so that a part, between the threading tube 32 and the clamping tube 34, of the traction wire 31 is threaded in the protective casing 34. In the process of bending or straightening the bendable section 11 of the tube body 10, the clamping tube 34 drives the traction wire 31 to move in the protective casing 33 and the threading tube 32, so that the part, between the threading tube 32 and the clamping tube 34, of the traction wire 31 is remained in the protective casing 33. The inner cavity of the protective casing 33 limits the degree of bending deformation of the traction wire 31, and can prevent the traction wire 31 from being severely bent or broken near the proximal end of the threading tube 32, thereby ensuring smooth implementation of the bend adjusting function of the bendable catheter 100.

## Claims

1. A bendable catheter (100), comprising:
a tube body (10) comprising a bendable section (11) arranged at a distal end thereof; and
a traction member (30), wherein the traction member (30) comprises a traction wire (31), a threading tube (32), a protective casing (33), and a clamping tube (34);
the traction wire (31) has a distal end connected to the bendable section (11), a proximal end wrapped and fixed within the clamping tube (34), and a remainder which is sequentially moveably threaded in the threading tube (32) and the protective casing (33) in a direction from the distal end of the tube body (10) to a proximal end of the tube body (10);
one end of the threading tube (32) close to the clamping tube (34) is fixed inside a distal end of the protective casing (33) without a gap in axial directions of the threading tube (32) and the protective casing (33) and without a relative movement between the threading tube (32) and the protective casing (33), and a distal end of the clamping tube (34) is movably threaded in the protective casing (33); and
the clamping tube (34) is operable to move in the protective casing (33) to drive the traction wire (31) to move in the protective casing (33) and the threading tube (32), so as to bend or straighten the bendable section (11).

2. The bendable catheter (100) of claim 1, wherein a proximal end of the threading tube (32) and the distal end of the protective casing (33) are in abutting joint, or the proximal end of the threading tube (32) is threaded in the distal end of the protective casing (33).

3. The bendable catheter (100) of claim 1 or 2, wherein
the protective casing (33) comprises an inclined section (332), a transition section (333), and a straight section (331) connected in sequence;
the transition section (333) is smoothly connected with the inclined section (332) and the straight section (331); and
the inclined section (332) is relatively fixed with a proximal end of the threading tube (32), a proximal end of the clamping tube (34) is movably threaded in the straight section (331).

4. The bendable catheter (100) of claim 1, wherein an inside diameter of the protective casing (33) is 0.1 mm to 0.3 mm greater than an outside diameter of the clamping tube (34), and is 0.3 mm to 0.6 mm greater than a diameter of the traction wire (31).

5. The bendable catheter (100) of claim 1, wherein a length of the protective casing (33) is greater than a distance that the traction wire (31) moves to drive the bendable section (11) to switch between a straight state and a state with a maximum bending angle.

6. The bendable catheter (100) of claim 1, wherein the proximal end of the threading tube (32) extends out of the tube body (10) and a remainder thereof is embedded in a wall of the tube body (10).

7. The bendable catheter (100) of claim 1, further comprising a bend adjusting handle (20), wherein the bend adjusting handle (20) is connected to the proximal end of the tube body (10) as well as the proximal end of the clamping tube (34), and configured to control the clamping tube (34) to move in the protective casing (33).

8. The bendable catheter (100) of claim 7, wherein
the bend adjusting handle (20) comprises a fixing base (21), a drive mechanism, and a drive control mechanism;
the proximal end of the tube body (10) is threaded and fixed in the fixing base (21);
the protective casing (33) is fixed relative to the fixing base (21);
the clamping tube (34) is connected to the drive mechanism; and
the drive control mechanism is connected to the drive mechanism and configured to control the drive mechanism to move relative to the fixing base (21), the drive mechanism is configured to move to drive the clamping tube (34) to move in the protective casing (33).

9. The bendable catheter (100) of claim 8, wherein
the drive mechanism comprises a slide block (221) and a fixed block (222) fixed to the slide block (221);
the clamping tube (34) is fixed to the fixed block (222);
the slide block (221) defines a threaded groove (2211) on a surface of the slide block (221);
the drive control mechanism comprises a rotary sleeve (231), the rotary sleeve (231) is provided with a thread engaged with the threaded groove (2211) on an inside of the rotary sleeve (231), and the rotary sleeve (231) is configured to rotate to drive the slide block (221) to move relative to the fixing base (21).

10. The bendable catheter (100) of claim 9, wherein a length of the protective casing (33) is greater than a maximum stroke of the slide block (221).

11. The bendable catheter (100) of claim 9, wherein
the fixing base (21) defines an axial slot (213) axially extending along the fixing base (21);
the slide block (221) is movably disposed in the axial slot (213); and
the rotary sleeve (231) is configured to rotate to drive the slide block (221) to move along the axial slot (213).

12. The bendable catheter (100) of claim 11, wherein
the proximal end of the tube body (10) is embedded in the fixing base (21);
a central axis of the tube body (10) coincides with a central axis of the fixing base (21); and
the fixing base (21) defines an inclined slot (214), the inclined slot (214) has one end extended to the tube body (10) and the other end extended to the axial slot (213), and the protective casing (33) is fixed in the inclined slot (214).

13. The bendable catheter (100) of claim 9, wherein the bend adjusting handle (20) further comprises a housing (50), the drive mechanism is accommodated in the housing (50), and a distal end of the rotary sleeve (231) is accommodated in the housing (50) and rotationally connected to the housing (50).

## Patentansprüche

1. Biegbarer Katheter (100), der Folgendes umfasst:
einen Rohrkörper (10), der einen an seinem distalen Ende angeordneten biegbaren Abschnitt (11) aufweist; und
ein Zugelement (30), wobei das Zugelement (30) einen Zugdraht (31), ein Gewinderohr (32), einen Schutzmantel (33) und ein Klemmrohr (34) umfasst;
der Zugdraht (31) hat ein distales Ende, das mit dem biegbaren Abschnitt (11) verbunden ist, ein proximales Ende, das innerhalb des Klemmrohrs (34) gewickelt und befestigt ist, und einen Rest, der sequentiell beweglich in das Gewinderohr (32) und den Schutzmantel (33) in einer Richtung vom distalen Ende des Rohrkörpers (10) zu einem proximalen Ende des Rohrkörpers (10) geschraubt ist;
ein Ende des Gewinderohrs (32) in der Nähe des Klemmrohrs (34) ist innerhalb eines distalen Endes des Schutzmantels (33) ohne einen Spalt in axialen Richtungen des Gewinderohrs (32) und des Schutzmantels (33) und ohne eine relative Bewegung zwischen dem Gewinderohr (32) und dem Schutzmantel (33) befestigt, und ein distales Ende des Klemmrohrs (34) ist beweglich in den Schutzmantel (33) eingeschraubt; und
das Klemmrohr (34) ist zum Bewegen im Schutzmantel (33) ausgelegt, um den Zugdraht (31) zum Bewegen in dem Schutzmantel (33) und dem Gewinderohr (32) anzutreiben, um den biegbaren Abschnitt (11) zu biegen oder zu richten.

2. Biegbarer Katheter (100) nach Anspruch 1, wobei ein proximales Ende des Gewinderohrs (32) und das distale Ende des Schutzmantels (33) aneinanderstoßen oder das proximale Ende des Gewinderohrs (32) in das distale Ende des Schutzmantels (33) eingeschraubt ist.

3. Biegsamer Katheter (100) nach Anspruch 1 oder 2, wobei
der Schutzmantel (33) einen geneigten Abschnitt (332), einen Übergangsabschnitt (333) und einen geraden Abschnitt (331) umfasst, die hintereinander geschaltet sind;
der Übergangsabschnitt (333) nahtlos mit dem geneigten Abschnitt (332) und dem geraden Abschnitt (331) verbunden ist; und
der geneigte Abschnitt (332) mit einem proximalen Ende des Gewinderohrs (32) relativ fest verbunden ist, ein proximales Ende des Klemmrohrs (34) beweglich in den geraden Abschnitt (331) eingeschraubt ist.

4. Biegbarer Katheter (100) nach Anspruch 1, wobei ein Innendurchmesser des Schutzmantels (33) 0.1 mm bis 0.3 mm größer ist als der Außendurchmesser des Klemmrohrs (34) und 0.3 mm bis 0.6 mm größer ist als der Durchmesser des Zugdrahts (31).

5. Biegbarer Katheter (100) nach Anspruch 1, wobei eine Länge des Schutzmantels (33) größer ist als eine Distanz, um die sich der Zugdraht (31) bewegt, um den biegbaren Abschnitt (11) zum Wechseln zwischen einem geraden Zustand und einem Zustand mit einem maximalen Biegewinkel anzutreiben.

6. Biegbarer Katheter (100) nach Anspruch 1, wobei das proximale Ende des Gewinderohrs (32) aus dem Rohrkörper (10) herausragt und ein Rest davon in eine Wand des Rohrkörpers (10) eingebettet ist.

7. Biegbarer Katheter (100) nach Anspruch 1, der ferner einen Biegeeinstellgriff (20) umfasst, wobei der Biegeeinstellgriff (20) sowohl mit dem proximalen Ende des Rohrkörpers (10) als auch mit dem proximalen Ende des Klemmrohrs (34) verbunden und zum Steuern des Klemmrohrs (34) zum Bewegen im Schutzmantel (33) konfiguriert ist.

8. Biegbarer Katheter (100) nach Anspruch 7, wobei
der Biegeeinstellgriff (20) eine Befestigungsbasis (21), einen Antriebsmechanismus und einen Antriebssteuermechanismus umfasst;
das proximale Ende des Rohrkörpers (10) in die Befestigungsbasis (21) eingeschraubt und dort befestigt ist;
der Schutzmantel (33) relativ zur Befestigungsbasis (21) befestigt ist;
das Klemmrohr (34) mit dem Antriebsmechanismus verbunden ist; und
der Antriebssteuermechanismus mit dem Antriebsmechanismus verbunden und zum Steuern des Antriebsmechanismus zum Bewegen relativ zur Befestigungsbasis (21) konfiguriert ist, wobei der Antriebsmechanismus so konfiguriert ist, dass er sich bewegt, um das Klemmrohr (34) zum Bewegen im Schutzmantel (33) anzutreiben.

9. Biegbarer Katheter (100) nach Anspruch 8, wobei
der Antriebsmechanismus einen Gleitblock (221) und einen am Gleitblock (221) befestigten festen Block (222) umfasst;
das Klemmrohr (34) an dem festen Block (222) befestigt ist;
der Gleitblock (221) eine Gewindenut (2211) auf einer Oberfläche des Gleitblocks (221) definiert,
der Antriebssteuermechanismus eine Drehhülse (231) umfasst, die Drehhülse (231) mit einem Gewinde in Eingriff mit der Gewindenut (2211) an einer Innenseite der Drehhülse (231) versehen ist und die Drehhülse (231) so konfiguriert ist, dass sie sich dreht, um den Gleitblock (221) zum Bewegen relativ zur Befestigungsbasis (21) anzutreiben.

10. Biegbarer Katheter (100) nach Anspruch 9, wobei eine Länge des Schutzmantels (33) größer ist als ein maximaler Hub des Gleitblocks (221).

11. Biegbarer Katheter (100) nach Anspruch 9, wobei
die Befestigungsbasis (21) einen axialen Schlitz (213) definiert, der sich axial entlang der Befestigungsbasis (21) erstreckt;
der Gleitblock (221) beweglich in dem axialen Schlitz (213) angeordnet ist; und
die Drehhülse (231) so konfiguriert ist, dass sie sich dreht, um den Gleitblock (221) zum Bewegen entlang des axialen Schlitzes (213) anzutreiben.

12. Biegbarer Katheter (100) nach Anspruch 11, wobei
das proximale Ende des Rohrkörpers (10) in der Befestigungsbasis (21) eingebettet ist;
eine zentrale Achse des Rohrkörpers (10) mit einer zentralen Achse der Befestigungsbasis (21) zusammenfällt; und
die Befestigungsbasis (21) einen geneigten Schlitz (214) definiert, der geneigte Schlitz (214) ein sich zum Rohrkörper (10) erstreckendes Ende hat und das andere Ende sich zum axialen Schlitz (213) erstreckt, und der Schutzmantel (33) in dem geneigten Schlitz (214) befestigt ist.

13. Biegbarer Katheter (100) nach Anspruch 9, wobei der Biegeeinstellgriff (20) ferner ein Gehäuse (50) umfasst, der Antriebsmechanismus in dem Gehäuse (50) untergebracht ist und ein distales Ende der Drehhülse (231) in dem Gehäuse (50) untergebracht und drehbar mit dem Gehäuse (50) verbunden ist.

## Revendications

1. Cathéter pouvant être courbé (100), comprenant:
un corps de tube (10) comprenant une section pouvant être courbée (11) agencée au niveau d'une extrémité distale de celui-ci; et
un élément de traction (30), dans lequel l'élément de traction (30) comprend un fil de traction (31), un tube d'enfilage (32), un boîtier protecteur (33), et un tube de serrage (34);
le fil de traction (31) a une extrémité distale connectée à la section pouvant être courbée (11), une extrémité proximale enveloppée et fixée au sein du tube de serrage (34), et un reste qui est enfilé séquentiellement de manière amovible dans le tube d'enfilage (32) et le boîtier protecteur (33) dans une direction allant de l'extrémité distale du corps de tube (10) à une extrémité proximale du corps de tube (10);
une extrémité du tube d'enfilage (32) proche du tube de serrage (34) est fixée à l'intérieur d'une extrémité distale du boîtier protecteur (33) sans écartement dans des directions axiales du tube d'enfilage (32) et du boîtier protecteur (33) et sans mouvement relatif entre le tube d'enfilage (32) et le boîtier protecteur (33), et une extrémité distale du tube de serrage (34) est enfilée de manière amovible dans le boîtier protecteur (33); et
le tube de serrage (34) peut être amené à fonctionner pour se déplacer dans le boîtier protecteur (33) pour entraîner le fil de traction (31) pour qu'il se déplace dans le boîtier protecteur (33) et le tube d'enfilage (32), de manière à courber ou à redresser la section pouvant être courbée (11).

2. Cathéter pouvant être courbé (100) selon la revendication 1, dans lequel une extrémité proximale du tube d'enfilage (32) et l'extrémité distale du boîtier protecteur (33) sont assemblées en butée, ou l'extrémité proximale du tube d'enfilage (32) est enfilée dans l'extrémité distale du boîtier protecteur (33).

3. Cathéter pouvant être courbé (100) selon la revendication 1 ou 2, dans lequel
le boîtier protecteur (33) comprend une section inclinée (332), une section de transition (333), et une section droite (331) connectées en séquence;
la section de transition (333) est connectée de manière régulière avec la section inclinée (332) et la section droite (331); et
la section inclinée (332) est relativement fixe avec une extrémité proximale du tube d'enfilage (32), une extrémité proximale du tube de serrage (34) est enfilée de manière amovible dans la section droite (331).

4. Cathéter pouvant être courbé (100) selon la revendication 1, dans lequel un diamètre intérieur du boîtier protecteur (33) est plus grand de 0.1 mm à 0.3 mm qu'un diamètre extérieur du tube de serrage (34), et est plus grand de 0.3 mm à 0.6 mm qu'un diamètre du fil de traction (31).

5. Cathéter pouvant être courbé (100) selon la revendication 1, dans lequel une longueur du boîtier protecteur (33) est supérieure à une distance parcourue par le fil de traction (31) pour entraîner la section pouvant être courbée (11) pour basculer entre un état droit et un état avec un angle de courbure maximal.

6. Cathéter pouvant être courbé (100) selon la revendication 1, dans lequel l'extrémité proximale du tube d'enfilage (32) s'étend hors du corps de tube (10) et le reste de celui-ci est incrusté dans une paroi du corps de tube (10).

7. Cathéter pouvant être courbé (100) selon la revendication 1, comprenant en outre une poignée d'ajustement de courbure (20), dans lequel la poignée d'ajustement de courbure (20) est connectée à l'extrémité proximale du corps de tube (10) ainsi qu'à l'extrémité proximale du tube de serrage (34), et configurée pour commander le tube de serrage (34) pour qu'il se déplace dans le boîtier protecteur (33).

8. Cathéter pouvant être courbé (100) selon la revendication 7, dans lequel
la poignée d'ajustement de courbure (20) comprend une base de fixation (21), un mécanisme d'entraînement, et un mécanisme de commande d'entraînement;
l'extrémité proximale du corps de tube (10) est enfilée et fixée dans la base de fixation (21);
le boîtier protecteur (33) est fixé par rapport à la base de fixation (21);
le tube de serrage (34) est connecté au mécanisme d'entraînement; et
le mécanisme de commande d'entraînement est connecté au mécanisme d'entraînement et configuré pour commander le mécanisme d'entraînement pour qu'il se déplace par rapport à la base de fixation (21), le mécanisme d'entraînement est configuré pour qu'il se déplace pour entraîner le tube de serrage (34) pour qu'il se déplace dans le boîtier protecteur (33).

9. Cathéter pouvant être courbé (100) selon la revendication 8, dans lequel
le mécanisme d'entraînement comprend un bloc de coulissement (221) et un bloc fixe (222) fixé au bloc de coulissement (221);
le tube de serrage (34) est fixé au bloc fixe (222);
le bloc coulissant (221) définit une rainure filetée (2211) sur une surface du bloc coulissant (221);
le mécanisme de commande d'entraînement comprend un manchon rotatif (231), le manchon rotatif (231) est pourvu d'un filet entré en prise avec la rainure filetée (2211) sur un intérieur du manchon rotatif (231), et le manchon rotatif (231) est configuré pour tourner pour entraîner le bloc coulissant (221) pour qu'il se déplace par rapport à la base de fixation (21).

10. Cathéter pouvant être courbé (100) selon la revendication 9, dans lequel une longueur du boîtier protecteur (33) est supérieure à une course maximale du bloc coulissant (221).

11. Cathéter pouvant être courbé (100) selon la revendication 9, dans lequel
la base de fixation (21) définit une fente axiale (213) s'étendant de manière axiale le long de la base de fixation (21);
le bloc coulissant (221) est disposé de manière mobile dans la fente axiale (213); et
le manchon rotatif (231) est configuré pour tourner pour entraîner le bloc coulissant (221) pour qu'il se déplace le long de la fente axiale (213).

12. Cathéter pouvant être courbé (100) selon la revendication 11, dans lequel
l'extrémité proximale du corps de tube (10) est incrustée dans la base de fixation (21);
un axe central du corps de tube (10) coïncide avec un axe central de la base de fixation (21); et
la base de fixation (21) définit une fente inclinée (214), la fente inclinée (214) a une extrémité étendue jusqu'au corps de tube (10) et l'autre extrémité étendue jusqu'à la fente axiale (213), et le boîtier protecteur (33) est fixé dans la fente inclinée (214).

13. Cathéter pouvant être courbé (100) selon la revendication 9, dans lequel la poignée d'ajustement de courbure (20) comprend en outre un logement (50), le mécanisme d'entraînement est accueilli dans le logement (50), et une extrémité distale du manchon rotatif (231) est accueillie dans le logement (50) et connectée en rotation au logement (50).
